# FASCICULE DE BREVET EUROPEEN

(11) **EP 0 358 574 B1**
(45) Date de publication et mention de la délivrance du brevet: **17.02.1993**
(21) Numéro de dépôt: 89402446.2
(22) Date de dépôt: 07.09.1989
(51) Int. Cl.: C07C 43/21, C07C 39/42, C07C 65/26, C07C 69/78, C07C 69/94, C07C 323/20, C07C 231/06

(54) **Procédé de préparation de dérivés de l'adamantane-1**
Verfahren und Herstellung von Adamantan-1-Derivaten
Process for the preparation of adamantane-1 derivatives

(30) Priorité: 07.09.1988 FR 8811706
(43) Date de publication de la demande: 14.03.1990
(73) Titulaire: CENTRE INTERNATIONAL DE RECHERCHES DERMATOLOGIQUES GALDERMA - CIRD GALDERMA, F-06560 Valbonne (FR)
(72) Inventeur: Pilgrim, William Robert, F-06560 Valbonne (FR); Lagiere, Joel, F-06560 Valbonne (FR)
(74) Mandataire: Stalla-Bourdillon, Bernard

(56) Documents cités:
- EP-A- 0 199 636
- US-A- 3 883 603
- JOURNAL OF MEDICINAL CHEMISTRY, vol. 18, no. 7, juillet 1975, pages 713-721, Washington, D.C., US; K. AIGAMI et al.: "Biologically active polycycloalkanes. 1. Antiviral adamantane derivatives"

## Description

La présente invention a pour objet un nouveau procédé de préparation de dérivés de l'adamantane-1.

Le radical adamantyl-1 est présent dans un certain nombre de composés actifs à action thérapeutique en particulier en tant que substituant de composés aromatiques.

Les méthodes de synthèse connues permettant d'accéder à ces composés font intervenir en tant que produit de départ un halogéno adamantane et plus particulièrement le chloro-1 adamantane ou le bromo-1 adamantane.

Ces procédés présentent l'inconvénient majeur d'un dégagement d'acide chlorhydrique ou bromhydrique en quantité importante. Il est toutefois possible de supprimer ces dégagements par addition au milieu réactionnel de sodium métallique ou d'une base forte aminée mais dans ce cas, l'on constate alors la formation de produits secondaires.

Il a également été proposé l'utilisation du trifluoroacétate d'adamantanol-1 en excès, sans catalyseur et sans solvant, mais cette méthode de synthèse n'est pas toujours reproductible et ne permet pas une généralisation à différents types de composés aromatiques.

Par ailleurs, tout comme le procédé faisant intervenir les halogénures d'adamantane, il se forme des produits secondaires notamment du type adamantane-1,3 disubstitué.

La présence de ces produits secondaires rend ces méthodes de synthèse particulièrement difficiles à mettre en oeuvre sur le plan industriel.

En effet, on ne peut envisager leur élimination par chromatographie et l'on doit donc procéder à des purifications par recristallisation, ce qui influe de façon non négligeable sur les coûts de production.

Par ailleurs, ces recristallisations ne permettent pas toujours d'éliminer la totalité des produits secondaires.

La présente invention propose un nouveau procédé de préparation de dérivés de l'adamantane-1, en particulier un procédé d'adamantylation de composés aromatiques, dans lequel la réaction est réalisée à température ambiante sans dégagement de gaz dangereux. Par ailleurs ce nouveau procédé réduit les temps de réaction et présente l'avantage capital de diminuer au maximum la formation de produits secondaires, voire même de les éliminer totalement.

Ce nouveau procédé selon l'invention permet d'obtenir les dérivés de l'adamantane-1 sous une forme très pure avec d'excellents rendements, dans la mesure où leur isolement sur le plan industriel peut être réalisé par addition d'un solvant organique hydroxylé, suivie d'une filtration et éventuellement d'un lavage aqueux.

Bien que le procédé selon la présente demande soit particulièrement recommandé pour l'adamantylation de composés aromatiques, il peut également être mis en oeuvre pour la préparation d'autres composés de la série de l'adamantane-1.

La présente invention a pour objet un procédé de préparation de dérivés de l'adamantane-1, ce procédé consistant à faire réagir un acyloxy-1 adamantane dont le radical acyle contient de 1 à 4 atomes de carbone, sur un composé récepteur, la réaction étant conduite en solution dans un solvant du type hydrocarbure aliphatique linéaire, ou cycloaliphatique, en présence d'acide sulfurique concentré, à température ambiante.

Selon l'invention, l'acyloxy-1 adamantane est de préférence le formyloxy-1 adamantane, l'acétoxy-1 adamantane ou le propionyloxy-1 adamantane.

Le solvant aliphatique linéaire, est de préférence l'hexane, l'heptane ou l'octane et le solvant cycloaliphatique est de préférence le cyclopentane, le cyclohexane ou le cyclooctane.

La proportion de solvant nécessaire pour la mise en oeuvre du procédé selon l'invention est généralement comprise entre 5 et 100 fois la quantité d'acyloxy-1 adamantane mise à réagir et la proportion d'acide sulfurique concentré est généralement comprise entre 0,1:1 et 0,5:1 par rapport à l'acyloxy-1 adamantane.

Selon une forme de réalisation préférée de l'invention, l'acyloxy-1 adamantane est préparé in situ par estérification de l'hydroxy-1 adamantane ou adamantanol-1 avec un anhydride d'acide en utilisant comme catalyseur l'acide sulfurique concentré.

L'acide carboxylique libéré durant la réaction d'estérification n'a aucune influence défavorable sur la conduite du procédé selon l'invention.

Le procédé selon l'invention est plus particulièrement destiné à l'adamantylation de composés aromatiques et dans ce cas le composé récepteur peut être par exemple l'anisole, le phénol, le toluène, le naphtalène, le thiophène, ou le furanne et leurs dérivés substitués.

Selon une forme de réalisation préférée, le composé récepteur aromatique est:
le bromo-4 anisole,
le bromo-4 phénol,
l'acide méthoxy-4 benzoïque,
le méthoxy-4 benzoate de méthyle,
le fluoro-2 méthoxy-4 benzoate de méthyle,
le fluoro-2 hydroxy-4 benzoate d'allyle,
l'(hydroxy-4 phényl)-6 naphtoate-2 de méthyle,
le (méthoxy-4 phényl)-6 naphtoate-2 de méthyle,
l'hydroxy-6 bromo-2 naphtalène,
le bromo-2 méthoxy-6 naphtalène.

Le composé récepteur peut être également un thiol et le procédé selon l'invention conduit alors à la formation d'un adamantyl thioéther. Parmi les thiols, on peut notamment mentionner le méthoxy-4 benzène thiol.

Le composé récepteur peut être également un nitrile tel que par exemple l'acétonitrile.

Dans ce cas le procédé selon l'invention conduit à la formation d'un amide qui peut ensuite être transformé selon les conditions conventionnelles, en amino-1 adamantane (ou 1-adamantanamine).

On va maintenant donner à titre d'illustration et sans aucun caractère limitatif plusieurs exemples de mise en oeuvre du procédé selon l'invention.

### EXEMPLE 1

### Préparation de l'(adamantyl-1)-2 bromo-4 méthoxy-1 benzène

### (a) A partir de l'acétoxy-1 adamantane

Dans un ballon tricol de 100ml placé sous azote, on verse 5g d'acétoxy-1 adamantane, 10ml de n-heptane et après dissolution complète, introduit, goutte à goutte, 1,26g d'acide sulfurique concentré. A 20°C, on verse 4,82g de bromo-4 anisole et on laisse agiter 24 heures. On rajoute 60ml d'éthanol dénaturé et on agite 2 heures. On filtre le solide sur verre fritté, on sèche à l'étuve sous vide à 20°C pendant 24 heures. On recueille 5,67g de produit brut attendu (F: 144-145°C).

### (b) A partir de l'adamantanol-1

Dans un ballon de 10 litres muni d'une agitation mécanique et d'un réfrigérant, sous azote, on place 750g d'adamantanol-1 et 2,4 l de n-heptane. Sous bonne agitation, on ajoute lentement 18,3g d'acide sulfurique concentré et ensuite 573,5g d'anhydride acétique. Durant l'addition la température passe de 21°C à 37°C environ. On laisse agiter pendant 15 heures à 21°C environ puis on introduit 241,5g d'acide sulfurique concentré. La température passe de 21°C à 28°C. Lorsque la température est revenue à 21°C, on introduit 921,3g de bromo-4 anisole et on laisse sous agitation pendant 24 heures. On ajoute 3 litres d'éthanol dénaturé et on agite une heure. On récupère le produit solide par filtration et on le lave sur le filtre avec 1 litre d'éthanol absolu. Après séchage à l'étuve sous vide à 25°C pendant 24 heures, on obtient 1,015kg du produit brut recherché (F: 142-145°C).

### EXEMPLE 2

### Préparation de l'(adamantyl-1)-2 bromo-4 phénol

Dans un ballon tricol de 100ml, sous azote, on place 1g d'acétoxy-1 adamantane et 10ml de n-heptane. Après dissolution complète, on introduit, goutte à goutte, 0,25g d'acide sulfurique concentré en maintenant la température à 20°C, puis on verse lentement 0,886g de bromo-4 phénol. Après avoir laissé 24 heures sous bonne agitation, on ajoute 20ml d'éthanol dénaturé en maintenant la température à 20°C. On évapore ensuite le solvant à sec sous pression réduite et on obtient un produit brut blanchâtre. On reprend ce produit dans l'eau à 60°C environ, on lave jusqu'à pH 6 et on sèche à l'étuve sous vide pendant 24 heures à 25°C. On recueille 1,24g de produit brut attendu (F: 140-141°C).

### EXEMPLE 3

### Préparation de l'acide (adamantyl-1)-3 méthoxy-4 benzoïque

Dans un ballon tricol de 100ml sous azote, on place 1g d'acétoxy-1 adamantane et 50ml de n-heptane. Après dissolution, on introduit, goutte à goutte, 0,25g d'acide sulfurique concentré, goutte à goutte, à une température de 22°C et verse lentement 0,783g d'acide méthoxy-4 benzoïque. Après avoir laissé 48 heures sous bonne agitation, on ajoute 50ml d'éthanol dénaturé et on filtre l'insoluble. Par concentration du filtrat au trois quart, un solide blanc précipite que l'on filtre sur verre fritté. On sèche à l'étuve sous vide pendant 24 heures à 30°C et on recueille 0,680g de produit brut attendu (F: 245-246°C).

### EXEMPLE 4

### Préparation de l'(adamantyl-1)-3 méthoxy-4 benzoate de méthyle

Dans un ballon tricol de 100ml, sous azote, on place 2g d'acétoxy-1 adamantane et 20ml de n-heptane. Après dissolution complète, on introduit, goutte à goutte, 0,5g d'acide sulfurique concentré. A une température de 20°C environ, on ajoute lentement 1,71g de méthoxy-4 benzoate de méthyle et on laisse agiter 48 heures. On filtre le solide obtenu sur verre fritté et on lave à l'eau jusqu'à neutralité. Après séchage à l'étuve sous vide pendant 24 heures à 25°C, on récupère 2g de produit brut attendu (F: 136-137°C).

### EXEMPLE 5

### Préparation de l'(adamantyl-1)-5 fluoro-2 méthoxy-4 benzoate de méthyle

Dans un ballon de 100ml, sous azote, on verse 2,48g d'adamantanol-1 et 10ml de n-heptane. On ajoute 0,034ml d'acide sulfurique concentré et 1,76ml d'anhydride acétique goutte à goutte. On laisse agiter 1 heure, ajoute 0,87ml d'acide sulfurique concentré puis une suspension de 2g de fluoro-2 méthoxy-4 benzoate de méthyle dans 10 ml de cyclohexane. Après 16 heures de réaction à température ambiante, on arrête l'agitation et soutire la phase supérieure. On évapore les solvants heptane et cyclohexane puis extrait le produit au dichlorométhane. La phase organique est lavée à l'eau bicarbonatée, séchée sur sulfate de magnésium et filtrée sur buchner. Après évaporation du solvant sous pression réduite à 40°C, le produit est dissous dans un mélange dichlorométhane / hexane (50/50) pour être ensuite purifié par filtration sur précouche de silice. Après passage d'1 litre d'éluant dichlorométhane / hexane (50/50), on évapore les solvants sous pression réduite à 30°C puis sèche le produit blanc obtenu à l'étuve ventilée à 50°C pendant 24 heures. On obtient 3g de produit pur recherché. (F : 82-88°C).

### EXEMPLE 6

### Préparation de l'(adamantyl-1)-3 fluoro-2 hydroxy-4 benzoate d'allyle

Dans un tricol de 250 ml, sous azote, on verse 3,04g d'adamantanol-1, 10ml de n-heptane et 63 µl d'acide sulfurique concentré. On ajoute goutte à goutte 2,16ml d'anhydride acétique et laisse agiter 3 heures à température ambiante. On additionne goutte à goutte 540 µl d'acide sulfurique concentré et on verse par portions 3,92g de fluoro-2 hydroxy-4 benzoate d'allyle. Après addition, on ajoute 25ml de dichlorométhane et agite à température ambiante pendant 24 heures. On évapore à sec, reprend le solide par de l'eau, neutralise à pH 7 avec du bicarbonate de sodium et extrait à l'éther éthylique. On lave la phase organique à l'eau puis à l'eau saturée en chlorure de sodium. On sèche sur sulfate de magnésium, filtre et évapore le filtrat. On obtient ainsi 7g de produit brut que l'on chromatographie sur colonne de silice éluée avec le dichlorométhane. Après trituration dans l'hexane, filtration et séchage en étuve ventilée à 50°C, on obtient 3,86g de produit attendu. (F : 219-222°C).

### EXEMPLE 7

### Préparation de l'[(adamantyl-1)-3 hydroxy-4 phényl]-6 naphtoate-2 de méthyle

Dans un ballon tricol de 100ml, sous azote, on place 1g d'acétoxy-1 adamantane et 40ml de n-heptane. Après dissolution complète, on introduit, goutte à goutte, 0,25g d'acide sulfurique concentré. A 20°C, on verse lentement 1,43g d'(hydroxy-4 phényl)-6 naphtoate-2 de méthyle et on laisse sous bonne agitation pendant 48 heures. Après addition de 40ml d'éthanol dénaturé et agitation pendant 2 heures, on filtre, lave le solide à l'heptane et sèche à l'étuve sous vide pendant 24 heures à 30°C. On récupère 1,47g de produit brut recherché (F: 255-256°C).

### EXEMPLE 8

### Préparation de l'[(adamantyl-1)-3 méthoxy-4 phényl]-6 naphtoate-2 de méthyle

Dans un ballon tricol de 100ml, sous azote, on place 1g d'acétoxy-1 adamantane et 50ml de cyclohexane. Après dissolution complète, on introduit, goutte à goutte, 0,25g d'acide sulfurique concentré et verse lentement 1g de (méthoxy-4 phényl)-6 naphtoate-2 de méthyle. Après avoir laissé 48 heures sous bonne agitation, on rajoute alors 20ml d'éthanol dénaturé et on agite pendant 2 heures. On filtre le solide obtenu sur verre fritté et lave abondamment à l'eau jusqu'à neutralité. Après séchage à 30°C pendant 24 heures à l'étuve sous vide, obtient 1g du produit brut recherché (F: 221-227°C).

### EXEMPLE 9

### Préparation de l'(adamantyl-1)-7 hydroxy-6 bromo-2 naphtalène

Dans un ballon de 100ml, sous azote, on place 2g d'acétoxy-1 adamantane, et 20ml de n-heptane et introduit, goutte à goutte, 0,5g d'acide sulfurique concentré. A une température de 22°C environ, on ajoute lentement 2,3g d'hydroxy-6 bromo-2 naphtalène et on laisse sous bonne agitation. On élimine le solvant par filtration et on reprend le résidu solide en suspension dans l'eau. On filtre, puis lave le résidu jusqu'à neutralité On lave à nouveau le solide rougeâtre obtenu à l'hexane jusqu'à obtention d'un filtrat incolore. Après séchage à l'étuve sous vide pendant 24 heures à 30°C, on obtient un produit brut que l'on chromatographie sur colonne de silice en utilisant comme éluant un mélange 1:9 d'acétate d'éthyle et d'hexane. On obtient après évaporation des solvants, 1,3g du produit brut recherché (F : 218-224°C).

### EXEMPLE 10

### Préparation de l'(adamantyl-1)-7 méthoxy-6 bromo-2 naphtalène

Dans un ballon de 100ml, sous azote, on place 1g d'acétoxy-1 adamantane et 30ml de n-heptane et introduit 0,25g d'acide sulfurique concentré. A 20°C environ, on verse lentement 1,22g de bromo-2 méthoxy-6 naphtalène et on laisse sous bonne agitation pendant 48 heures. On introduit 20ml d'éthanol dénaturé et évapore à sec sous pression réduite. On reprend dans un minimum d'éthanol dénaturé et on filtre le précipité sur verre fritté. Après séchage du produit brut à l'étuve sous vide à 30°C pendant 24 heures on purifie par chromatographie sur colonne de silice pour obtenir 0,4g du produit brut recherché (F: 164-168°C).

### EXEMPLE 11

### Préparation de l'(adamantyl-1)-2 hydroxy-1 naphtalène

Dans un ballon tricol de 100ml, sous azote, on place 2,7g d'acétoxy-1 adamantane et 5ml de cyclohexane. Après dissolution complète, on introduit 1,4g d'acide sulfurique concentré. On ajoute, en une seule fois, une suspension de 2g de naphtol-1 dans 15ml de cyclohexane et on laisse agiter pendant 45 minutes. On ajoute 20ml d'éthanol et on filtre. On lave le résidu à l'éthanol et à l'eau puis on le sèche sur le filtre. Après recristallisation dans le cyclohexane, on obtient 0,8g du produit recherché (F : 209,2 - 209,6°C).

### EXEMPLE 12

### Préparation de l'(adamantyl-1) méthoxy-4 phényl sulfure

Dans un ballon tricol de 100ml, sous azote, on place 1g d'acétoxy-1 adamantane et 20ml de n-heptane. Après dissolution complète, on introduit, goutte à goutte, 0,25g d'acide sulfurique concentré. A 22°C environ, on additionne à l'aide d'une seringue 0,63ml de méthoxy-4 benzènethiol et on laisse sous bonne agitation pendant 24 heures. On introduit alors 20ml d'éthanol dénaturé, concentre sous pression réduite à 40°C pour obtenir un produit brut que l'on reprend à l'eau. On extrait alors le produit au dichlorométhane et lave la phase organique à l'eau jusqu'à neutralité. Après évaporation de la phase organique, on chromatographie le résidu sur colonne de silice en utilisant comme éluant un mélange 1:4 de dichlorométhane et d'hexane. Après évaporation des solvants on obtient ainsi 1g du produit brut recherché (F : 70-72°C).

### EXEMPLE 13

### Préparation de l'(adamantyl-1) N-acétamide

Dans un ballon de 50ml, sous azote, on verse 1g d'acétoxy-1 adamantane et 10ml de n-heptane. Après dissolution complète, on introduit goutte à goutte 0,2ml d'acide sulfurique concentré. A une température de 20°C environ, on additionne goutte à goutte 0,27ml d'acétonitrile et laisse sous agitation pendant 24 heures. Après filtration sur verre fritté, on récupère un solide blanc qui est ensuite mis en suspension dans 20ml d'eau déminéralisée. Après agitation pendant 1 heure à température ambiante, le produit est filtré et séché en étuve ventilée à 60°C pendant 24 heures. On récupère 450mg de produit attendu . (F : 148-150°C).

## Revendications

1. Procédé de préparation de dérivés de l'adamantane-1, caractérisé par le fait qu'il consiste à faire réagir un acyloxy-1 adamantane dont le radical acyle contient de 1 à 4 atomes de carbone, sur un composé récepteur, dans un solvant du type hydrocarbure aliphatique linéaire, ou cycloaliphatique, en présence d'acide sulfurique concentré, à température ambiante.

2. Procédé selon la revendication 1, caractérisé par le fait que l'acyloxy-1 adamantane est le formyloxy-1 adamantane, l'acétoxy-1 adamantane ou le propionyloxy-1 adamantane.

3. Procédé selon la revendication 1, caractérisé par le fait que le solvant aliphatique linéaire, est l'hexane, l'heptane ou l'octane, de préférence l'heptane.

4. Procédé selon la revendication 1, caractérisé par le fait que le solvant cycloaliphatique est le cyclopentane, le cyclohexane ou le cyclooctane.

5. Procédé selon l'une quelconque des revendications 1 à 4, caractérisé par le fait que le solvant est utilisé en une proportion comprise entre 5 et 100 fois la quantité d'acyloxy-1 adamantane.

6. Procédé selon la revendication 1, caractérisé par le fait que l'acide sulfurique concentré est utilisé en une proportion comprise entre 0,1:1 et 0,5:1 de la quantité d'acyloxy-1 adamantane.

7. Procédé selon l'une quelconque des revendications 1 à 6, caractérisé par le fait que le composé récepteur est un composé aromatique, pris dans le groupe constitué par l'anisole, le phénol, le toluène, le naphtalène, le thiophène ou le furanne et leurs dérivés substitués.

8. Procédé selon la revendication 7, caractérisé par le fait que le composé récepteur aromatique est:
le bromo-4 anisole,
le bromo-4 phénol,
l'acide méthoxy-4 benzoïque,
le méthoxy-4 benzoate de méthyle
le fluoro-2 méthoxy-4 benzoate de méthyle,
le fluoro-2 hydroxy-4 benzoate d'allyle,
l'(hydroxy-4 phényl)-6 naphtoate-2 de méthyle,
le (méthoxy-4 phényl)-6 naphtoate-2 de méthyle,
l'hydroxy-6 bromo-2 naphtalène,
le bromo-2 méthoxy-6 naphtalène.

9. Procédé selon l'une quelconque des revendications 1 à 6, caractérisé par le fait que le composé récepteur est le méthoxy-4 benzène thiol.

10. Procédé selon l'une quelconque des revendications 1 à 6, caractérisé par le fait que le composé récepteur est l'acétonitrile.

## Patentansprüche

1. Verfahren zur Herstellung von 1-Adamantanderivaten, dadurch gekennzeichnet, daß man ein 1-Acyloxyadamantan in dem der Acylrest 1 bis 4 Kohlenstoffatome enthält, mit einer Rezeptorverbindung in einem Lösungsmittel vom Typ cycloaliphatischer oder linearer aliphatischer Kohlenwasserstoff in Gegenwart konzentrierter Schwefelsäure bei Raumtemperatur umsetzt.

2. Verfahren nach Anspruch 1, dadurch gekennzeichnet, daß das 1-Acyloxyadamantan 1-Formyloxyadamantan, 1-Acetoxyadamantan oder 1-Propionyloxyadamantan ist.

3. Verfahren nach Anspruch 1, dadurch gekennzeichnet, daß das aliphatische lineare Lösungsmittel Hexan, Heptan oder Octan, vorzugsweise Heptan, ist.

4. Verfahren nach Anspruch 1, dadurch gekennzeichnet, daß das cycloaliphatische Lösungsmittel Cyclopentan, Cyclohexan oder Cyclooctan ist.

5. Verfahren nach einem der Ansprüche 1 bis 4, dadurch gekennzeichnet, daß das Lösungsmittel in einem Anteil zwischen 5 und 100 mal der Menge des 1-Acyloxyadamantans verwendet wird.

6. Verfahren nach Anspruch 1, dadurch gekennzeichnet, daß die konzentrierte Schwefelsäure in einer Menge zwischen 0,1 : 1 und 0,5 : 1 von der Menge des 1-Acyloxyadamantans verwendet wird.

7. Verfahren nach einem der Ansprüche 1 bis 6, dadurch gekennzeichnet, daß die Rezeptorverbindung eine aromatische Verbindung, ausgewählt aus der Gruppe bestehend aus Anisol. Phenol, Toluol, Naphthalin, Thiophen oder Furan und ihren substituierten Derivaten ist.

8. Verfahren nach Anspruch 7, dadurch gekennzeichnet, daß die aromatische Rezeptorverbindung eine der folgenden ist:
4-Bromanisol,
4-Bromphenol,
4-Methoxybenzoesäure,
4-Methoxymethylbenzoat,
2-Fluor-2-methoxy-4-methylbenzoat,
2-Fluor-2-hydroxy-4-Allylbenzoat,
6-(4-Hydroxyphenyl)-2-methylnaphthoat,
6-(4-Methoxyphenyl)-2-methylnaphthoat,
6-Hydroxy-2-brom-naphthalin,
2-Brom-6-methoxynaphthalin.

9. Verfahren nach einem der Ansprüche 1 bis 6, dadurch gekennzeichnet, daß die Rezeptorverbindung 4-Methoxy-benzolthiol ist.

10. Verfahren nach einem der Ansprüche 1 bis 6, dadurch gekennzeichnet, daß die Rezeptorverbindung Acetonitrill ist.

## Claims

1. Process for the preparation of 1-adamantane derivatives, characterised in that it consists in reacting a 1-(acyloxy)adamantane, the acyl radical of which contains from 1 to 4 carbon atoms, with a receptor compound in a solvent of the linear aliphatic or cycloaliphatic hydrocarbon type in the presence of concentrated sulphuric acid at room temperature.

2. Process according to Claim 1, characterised in that the 1-(acyloxy)adamantane is 1-(formyloxy)adamantane, 1-(acetoxy)adamantane or 1-(propionyloxy)adamantane.

3. Process according to Claim 1, characterised in that the linear aliphatic solvent is hexane, heptane or octane, preferably heptane.

4. Process according to Claim 1, characterised in that the cycloaliphatic solvent is cyclopentane, cyclohexane or cyclooctane.

5. Process according to any one of Claims 1 to 4, characterised in that the solvent is used in a proportion of between 5 and 100 times the quantity of 1-(acyloxy)adamantane.

6. Process according to Claim 1, characterised in that the concentrated sulphuric acid is used in a proportion of between 0.1:1 and 0.5:1 of the quantity of 1-(acyloxy)adamantane.

7. Process according to any one of Claims 1 to 6, characterised in that the receptor compound is an aromatic compound, taken from the group consisting of anisole, phenol, toluene, naphthalene, thiophene or furan and their substituted derivatives.

8. Process according to Claim 7, characterised in that the aromatic receptor compound is:
4-bromoanisole,
4-bromophenol,
4-methoxybenzoic acid,
methyl 4-methoxybenzoate,
methyl 2-fluoro-4-methoxybenzoate,
allyl 2-fluoro-4-hydroxybenzoate,
methyl 6-(4-hydroxyphenyl)-2-naphthoate,
methyl 6-(4-methoxyphenyl)-2-naphthoate,
6-hydroxy-2-bromonaphthalene,
2-bromo-6-methoxynaphthalene.

9. Process according to any one of Claims 1 to 6, characterised in that the receptor compound is 4-methoxybenzenethiol.

10. Process according to any one of Claims 1 to 6, characterised in that the receptor compound is acetonitrile.
